# EUROPEAN PATENT SPECIFICATION

(11) **EP 1 854 493 B1**
(45) Date of publication and mention of the grant of the patent: **23.12.2015**
(21) Application number: 07108080.8
(22) Date of filing: 11.05.2007
(51) Int. Cl.: A61M 16/08, A61M 16/00, A61M 16/10, A61M 16/20, A61M 16/06

(54) **Improvements relating to gas delivery apparatus**
Verbesserungen im Zusammenhang mit Gaszuführungsvorrichtungen
Améliorations associées à un appareil de fourniture de gaz

(30) Priority: 12.05.2006 GB 0609401
(43) Date of publication of application: 14.11.2007
(73) Proprietor: Intersurgical AG, Vaduz (LI)
(72) Inventor: Bowsher, Francis Richard, Reading, Berkshire RG41 7DB (GB)
(74) Representative: Adamson Jones

(56) References cited:
- EP-A- 1 582 231
- WO-A-2005/030334
- US-A- 2 535 938
- US-A- 3 182 659
- US-A- 3 859 997
- US-A- 5 492 114
- US-A1- 2003 196 664

## Description

This invention relates to respiratory gas delivery apparatus, and in particular to the connection between a respiratory mask and a reservoir bag assembly of such apparatus.

Patients suffering from conditions such as hypoxemia, severe trauma, and acute myocardial infarction, are typically provided with supplemental oxygen for inhalation. A widely-used method for delivering supplemental oxygen to a patient is to use a respiratory mask.

Simple respiratory masks generally fit over a patient's nose and mouth, and are secured around the patient's head by an elastic strap. A respiratory mask as mentioned in the preamble of claim 1 is disclosed in EP1582231 A1.

Oxygen is delivered through a small-bore tube connected to the base of the mask, and openings on each side of the mask enable escape of exhaled gases and also serve as atmospheric air entrainment ports. The Fraction of Inspired Oxygen (FIO₂) varies with the patient's inspiratory flow rate and respiratory pattern, as well as mask fit. In order to deliver a higher concentration of oxygen to a patient, high concentration respiratory masks including a reservoir bag are typically used. In particular, high concentration respiratory masks generally include a reservoir bag that is charged with oxygen during use, so that sufficient oxygen is available to meet peak inspiratory flow demands of the patient.

Conventionally, respiratory masks and reservoir bag assemblies are manufactured and supplied as separate components, which are then connected together before use. In particular, conventional reservoir bag assemblies comprise a tubular connector that is adapted to engage a corresponding tubular connector of a respiratory mask. However, this arrangement suffers from the major disadvantage that the reservoir bag assembly is liable to become detached during use.

There has now been devised an improved reservoir bag assembly, an improved respiratory mask, and improved respiratory gas delivery apparatus, that overcome or substantially mitigate the above-mentioned and/or other disadvantages associated with the prior art.

According to a first aspect of the invention, there is provided respiratory gas delivery apparatus comprising a respiratory mask and a reservoir bag assembly, the respiratory mask comprising a mask body defining a cavity and being adapted to fit about the mouth and/or nose of the patient such that respiratory gas can be inhaled by the patient from the cavity, and an inhalation port for enabling passage of gas into the cavity of the mask body, and the reservoir bag assembly comprising a flexible bag for containing a reservoir of respiratory gas, the respiratory mask and the reservoir bag assembly being provided with corresponding connectors adapted for engagement so as to enable fluid communication between the flexible bag and the respiratory mask, wherein said connectors are provided with formations adapted to interlock upon engagement of the reservoir bag assembly with the respiratory mask.

According to a further aspect of the invention, there is provided a reservoir bag assembly for use with a respiratory mask, the reservoir bag assembly comprising a flexible bag for containing a reservoir of respiratory gas, and a connector adapted for engagement with a corresponding connector of the respiratory mask so as to enable fluid communication between the flexible bag and the respiratory mask, wherein the connector of the reservoir bag assembly includes formations adapted to interlock with corresponding formations of the connector of the respiratory mask upon engagement of the reservoir bag assembly with the respiratory mask.

According to a further aspect of the invention, there is provided a respiratory mask for use with a reservoir bag assembly, the respiratory mask comprising a mask body defining a cavity and being adapted to fit about the mouth and/or nose of the patient such that respiratory gas can be inhaled by the patient from the cavity, an inhalation port for enabling passage of gas into the cavity of the mask body, and a connector adapted for engagement with a corresponding connector of the reservoir bag assembly so as to enable fluid communication between the inhalation port and the reservoir bag assembly, wherein the connector of the respiratory mask includes formations adapted to interlock with corresponding formations of the connector of the reservoir bag assembly upon engagement of the respiratory mask with the reservoir bag assembly.

The respiratory gas delivery apparatus, reservoir bag assembly and respiratory mask according to the invention are advantageous principally because the interlocking formations of the reservoir bag assembly and the respiratory mask significantly reduce the risk of the reservoir bag assembly becoming detached from the respiratory mask during use.

The interlocking formations of one component of the respiratory gas delivery apparatus, ie either the respiratory mask or the reservoir bag assembly, preferably comprise a retaining arm adapted to engage a corresponding detent of the other component, such that the retaining arm and the corresponding detent act to prevent disengagement of the corresponding connectors. Most preferably, the retaining arm is resilient in form so that the retaining arm is resiliently biased into engagement with the detent.

The respiratory mask and the reservoir bag assembly may each comprise one or more retaining arms and/or one or more detents. Most preferably, however, either the respiratory mask or the reservoir bag assembly comprises a plurality of retaining arms adapted to engage one or more corresponding detents of the other component.

The interlocking formations of the reservoir bag assembly and the respiratory mask are preferably brought into interlocking engagement by relative movement of the corresponding connectors during engagement of those connectors. Where the interlocking formations require the connectors to have a particular orientation relative to each other, the connectors preferably include guide formations that limit the relative orientation of the connectors during engagement.

Each retaining arm is preferably adapted to be deformed from a rest configuration during engagement of the corresponding connectors, most preferably by means of a guide surface of the corresponding connector. The guide surface is preferably adapted so that the deformed retaining arm then travels along the guide surface until the retaining arm is able to resiliently engage the corresponding detent. In particular, the deformed retaining arm preferably travels along the guide surface during engagement of the connectors until corresponding operative surfaces of the retaining arm and the detent are aligned. Each deformed retaining arm preferably has sufficient resilience that when the operative surfaces are aligned, the deformed retaining arm is able to move back towards its rest configuration, such that the operative surfaces are brought into engagement. The operative surfaces are preferably orientated so that disengagement of the corresponding connectors is prevented by the interlocking engagement of the corresponding retaining arm(s) and detent(s).

The corresponding operative surfaces are each preferably orientated generally perpendicularly relative to the direction along which the connectors are moved relative to each other during engagement. These operative surfaces are preferably adapted so that engagement of corresponding operative surfaces acts to prevent disengagement of the corresponding connectors.

In presently preferred embodiments, the interlocking formations are defined by a cross-member that extends across the interior of each connector, and which faces the port from which that connector extends. In this case, one or both of the connectors may include one or more recesses for accommodating the cross-member of the corresponding connector in the engaged configuration of the connectors.

In presently preferred embodiments, the interlocking formations of either the respiratory mask or the reservoir bag assembly comprise a plurality of retaining arms that extend from the associated cross-member, and are orientated generally parallel to the direction along which the connectors are moved relative to each other during engagement. Each retaining arm preferably includes an enlarged head that defines the operative surface of that retaining arm. In this case, the corresponding component, ie either the respiratory mask or the reservoir bag assembly, preferably comprises a plurality of detents, each having an operative surface that is situated adjacent to a guide surface of the associated cross-member. In use, the enlarged head of each retaining arm is deformed by the guide surface of the corresponding detent, such that the enlarged head slides along the guide surface until the operative surface of the enlarged head is aligned with the operative surface of the corresponding detent. Each deformed retaining arm preferably has sufficient resilience that when the operative surfaces are aligned, the deformed retaining arm returns towards its rest configuration, such that the operative surfaces are brought into engagement.

Furthermore, the detents are preferably defined by a pair of ledges on each side of an opening in the associated cross-member, and the corresponding retaining arms are preferably arranged with one pair of adjacent retaining arms at each end of the associated cross-member. The pair of ledges preferably define operative surfaces on each side of the central opening, and guide surfaces are defined by the walls of the central opening. In this case, in use, the enlarged heads of each pair of adjacent retaining arms are preferably deformed towards each other and guided along said guide surfaces until the operative surfaces of the enlarged heads are aligned with the operative surfaces of the corresponding detents. The deformed retaining arms preferably then return to their rest configurations such that the operative surfaces of the interlocking formations are brought into engagement.

Most preferably, a surface of the retaining arm and/or the guide surface is inclined in an appropriate manner relative to the direction of motion of the connectors during engagement to bring about the desired deformation of the retaining arm. In the case of retaining arms having enlarged heads, the enlarged heads preferably have tapered ends.

Since respiratory gas delivery apparatus comprising a respiratory mask and a reservoir bag assembly is generally adapted for disposal after use, there is no need for the respiratory mask and reservoir bag assembly to be disengageable from each other. For this reason, the interlocking formations of the respiratory mask and the reservoir bag assembly may be adapted such that disengagement of these components is not possible without breaking those formations.

The respiratory mask and/or the reservoir bag assembly preferably therefore includes a locking member that prevents disengagement of the interlocking formations, for instance by preventing deformation of the one or more retaining arms out of engagement with the corresponding one or more detents. In particular, the locking member preferably includes one or more resilient arms that are adapted to be deformed away from a rest configuration by a retaining arm that has itself been deformed during the engagement action. The one or more resilient arms of the locking member are preferably adapted to return to their rest configuration when the deformed retaining arm moves into engagement with a corresponding detent, and hence no longer acts upon the one or more resilient arms. Most preferably, the one or more resilient arms then become situated adjacent to the previously deformed retaining arm so as to prevent disengagement of the retaining arm from the corresponding detent.

The flexible bag of the reservoir bag assembly may be formed of any suitable material, that is to say any material which is impermeable to gas, and which is flexible enough to expand and contract during use. However, the flexible bag is most preferably formed of a synthetic plastics material, such as polyvinylchloride (PVC). Most preferably, the flexible bag is formed by welding or otherwise joining two sheets of such material.

The connectors of the respiratory mask and the reservoir bag assembly preferably each include a tubular connecting member. The tubular connecting members are preferably adapted to engage each other so as to define a fluid conduit between the inhalation port of the respiratory mask and the flexible bag of the reservoir bag assembly. Most preferably, one of the tubular connecting members is adapted to receive the other tubular connecting member with a sufficiently close fit for the interface between the connecting members to be hermetically sealed.

In presently preferred embodiments, the connector of the reservoir bag assembly includes a tubular connecting member that is adapted to receive a tubular connector member of the respiratory mask. The tubular connecting members are preferably both generally cylindrical in shape, and preferably have a similar length.

The interlocking formations of the respiratory mask and the reservoir bag assembly are preferably accommodated within their associated tubular connecting members, so that the interlocking formations are brought into interlocking engagement as one tubular connecting member is received within the other tubular connecting member.

The connector of the reservoir bag assembly preferably also includes a mount to which the flexible bag is bonded. The mount preferably has the form of a continuous loop so that it defines an entrance port, and most preferably has the form of a flange. In particular, the flexible bag is preferably bonded to the mount, for instance by welding, so that the mount defines an entrance port leading into the interior of the flexible bag. Most preferably, the flexible bag is hermetically sealed, save for the opening defined by the entrance port.

The mount may be adapted to provide protection for parts of the respiratory mask that are particularly liable to be damaged and/or accidentally occluded during use. In presently preferred embodiments, the mount is adapted to cover, but be separated from, one or more safety ports of the respiratory mask. The one or more safety ports are preferably adapted to enable the passage of atmospheric air into the cavity of the mask body in the event of a failure of the supply of respiratory gas to the cavity of the respiratory mask.

The tubular connecting member of the reservoir bag assembly preferably extends from the entrance port defined by the mount, and the tubular connecting member of the respiratory mask preferably extends from the inhalation port, such that a fluid conduit is defined by the tubular connecting members in an engaged configuration between the entrance port and the inhalation port.

The connector of the reservoir bag assembly is preferably also adapted for engagement with a corresponding connector of a reservoir gas supply conduit, so as to provide fluid communication between the reservoir gas supply conduit and the reservoir bag assembly. In particular, the connector of the reservoir bag assembly preferably includes a tubular inlet adapted for engagement with a corresponding tubular connector of a reservoir gas supply conduit, and is preferably also provided with a fluid conduit extending between the tubular inlet and the entrance port of the flexible bag.

In presently preferred embodiments, the entrance port defined by the mount includes a partition such that the entrance port comprises two separate apertures. In this case, the tubular connecting member that is adapted for connection with the connector of the respiratory mask extends from a first aperture, and the fluid conduit that leads to the tubular inlet extends from a second aperture.

The respiratory gas delivery apparatus preferably includes a respiratory gas supply conduit that is adapted at one end for connection to a supply of respiratory gas, and at the other end for connection to the inhalation port of the respiratory mask. In particular, the respiratory gas supply conduit preferably includes a tubular connector at each end.

The mask body is preferably arranged so that the cavity accommodates the patient's nose and mouth when fitted to the patient. Most preferably, the mask body comprises a mouth portion and a nose portion, the depth of the cavity defined by the nose portion being greater then the depth of the cavity defined by the mouth portion.

The respiratory mask preferably includes one or more sealing components extending from the peripheral edge of the mask body and defining a contact surface that contacts the face of the patient during use. The contact surface preferably extends along the entire peripheral edge of the mask body so as to seal the cavity against the face of a patient during use. Each sealing component preferably comprises one or more sealing members that define the contact surface. Each sealing member is preferably formed of a resilient material so that it deforms into conformity with the contours of a patient's face during use. In particular, each sealing member is preferably formed of an elastomeric material.

The one or more sealing components are preferably formed from an elastomeric material, which is most preferably a Styrene-Ethylene-Butylene-Styrene (SEBS)-based thermoplastic elastomer. In this case, the respiratory mask is preferably manufactured using a so-called two-shot injection moulding process. In particular, the mask body is preferably injection moulded as a single component of a relatively rigid material, and the elastomeric material of the respiratory mask is then preferably injection moulded onto the surface of the mask body. The mask body and the elastomeric parts of the respiratory mask are bonded together by this process.

In any case, the mask body is preferably formed as a single component of a relatively rigid material. In particular, the mask body is preferably formed so that it maintains its shape when subjected to normal handling, packaging and storage conditions. The mask body is preferably formed from plastics material in an injection moulding process. Most preferably, the mask body is formed of polypropylene. In addition, in presently preferred embodiments, the mask body is at least partially transparent so that the patient is visible through the mask body during use.

Most preferably, the inhalation port and the connector of the respiratory mask are formed integrally with the mask body, such that the mask body including the inhalation port and the connector is defined by a single component. In particularly preferred embodiments, the mask body including the inhalation port and the connector is formed as a single component from plastics material in an injection moulding process. Similarly, the connector of the respiratory bag assembly is preferably formed as a single component from plastics material in an injection moulding process.

In order to reduce the risk of the flexible bag collapsing about the entrance port, and hence causing severe restriction or prevention of the passage of gas through that port, the connector of the reservoir bag assembly and/or the connector of the respiratory mask preferably includes one or more safety projections that project beyond the entrance port into the flexible bag and at least partially define a discontinuous surface upon which the flexible bag rests when collapsed. These safety formations ensure that gas is able to pass between the interior of the flexible bag and the entrance port in the event that the flexible bag has collapsed across the entrance port, during use.

Hence, according to a further aspect of the invention, there is provided respiratory gas delivery apparatus comprising a respiratory mask and a reservoir bag assembly, the respiratory mask comprising a mask body defining a cavity and being adapted to fit about the mouth and/or nose of the patient such that respiratory gas can be inhaled by the patient from the cavity, and an inhalation port for enabling passage of gas into the cavity of the mask body, and the reservoir bag assembly comprising a flexible bag for containing a reservoir of respiratory gas, the respiratory mask and the reservoir bag assembly being provided with corresponding connectors adapted for engagement so as to enable fluid communication between the flexible bag and the respiratory mask through an entrance port of the flexible bag, wherein either the connector of the reservoir bag assembly or the connector of the respiratory mask includes one or more safety projections that project beyond the entrance port into the flexible bag and at least partially define a discontinuous surface upon which the flexible bag rests when collapsed.

According to a further aspect of the invention, there is provided a reservoir bag assembly for use with a respiratory mask, the reservoir bag assembly comprising a flexible bag for containing a reservoir of respiratory gas, and a connector adapted for engagement with a corresponding connector of the respiratory mask so as to enable fluid communication between the flexible bag and the respiratory mask through an entrance port of the flexible bag, wherein the connector of the reservoir bag assembly includes one or more safety projections that project beyond the entrance port into the flexible bag and at least partially define a discontinuous surface upon which the flexible bag rests when collapsed.

According to a further aspect of the invention, there is provided a respiratory mask for use with a reservoir bag assembly, the respiratory mask comprising a mask body defining a cavity and being adapted to fit about the mouth and/or nose of the patient such that respiratory gas can be inhaled by the patient from the cavity, an inhalation port for enabling passage of gas into the cavity of the mask body, and a connector adapted for engagement with a corresponding connector of the reservoir bag assembly so as to enable fluid communication between the inhalation port and a flexible bag of the reservoir bag assembly through an entrance port of the flexible bag, wherein the connector of the respiratory mask includes one or more safety projections that project beyond the entrance port into the flexible bag and at least partially define a discontinuous surface upon which the flexible bag rests when collapsed.

The reservoir bag assembly and the respiratory mask preferably have a similar form to that described above in relation to other aspects of the invention. In particular the connectors of the reservoir bag assembly and the respiratory mask preferably each include a tubular connecting member, and the corresponding tubular connecting members are preferably adapted to engage each other so as to define a fluid conduit between the inhalation port and the entrance port. In this case, the safety projections of the reservoir bag assembly and/or the respiratory mask preferably extend from the end of the associated tubular connecting member that defines, and/or is situated adjacent to, the entrance port. The safety projections preferably have rounded end surfaces so as to reduce the risk of the flexible bag becoming damaged by the safety projections during use.

Preferred embodiments of the invention will now be described in greater detail, by way of illustration only, with reference to the accompanying drawings, in which
Figure 1 is a perspective view of first embodiment of a respiratory gas delivery apparatus according to the invention;
Figure 2 is a first perspective view of a respiratory mask according to the invention, which forms part of the apparatus shown in Figure 1;
Figure 3 is a second perspective view of the respiratory mask of Figure 2;
Figure 4 is a first perspective view of a valve assembly that forms part of the respiratory mask shown in Figures 2 and 3;
Figure 5 is a second perspective view, partly cut-away, of the valve assembly of Figure 4;
Figure 6 is a perspective view of the valve assembly of Figures 4 and 5, with a valve membrane that forms part of the valve assembly having been removed;
Figure 7 is a perspective view of the valve membrane in isolation;
Figure 8 is a first fragmentary perspective view of a reservoir bag connector attached to the respiratory mask, which both form part of the apparatus according to the invention;
Figure 9 is a second fragmentary perspective view, partly cut-away, of the reservoir bag connector attached to the respiratory mask;
Figure 10 is a third fragmentary perspective view, partly cut-away, of the reservoir bag connector attached to the respiratory mask;
Figure 11 is a cross-sectional view of the reservoir bag connector attached to the respiratory mask;
Figure 12 is a first perspective view of a valve assembly that forms part of a second embodiment of a respiratory mask according to the invention; and
Figure 13 is a cross-sectional view of a part of the valve assembly of Figure 12.

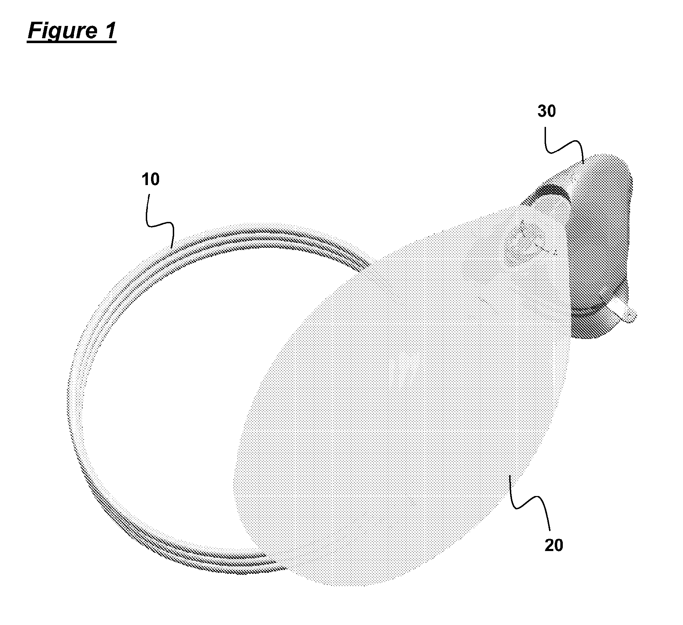

Figure 1 shows respiratory gas delivery apparatus according to the invention comprising a small-bore supply tube 10, a reservoir bag assembly 20, and a respiratory mask 30.

The small-bore supply tube 10 includes a tubular connector (only partially visible in Figure 1) at each end, so that it is adapted for connection to a supply of respiratory gas at one end, and to the reservoir bag assembly 20 at the other end.

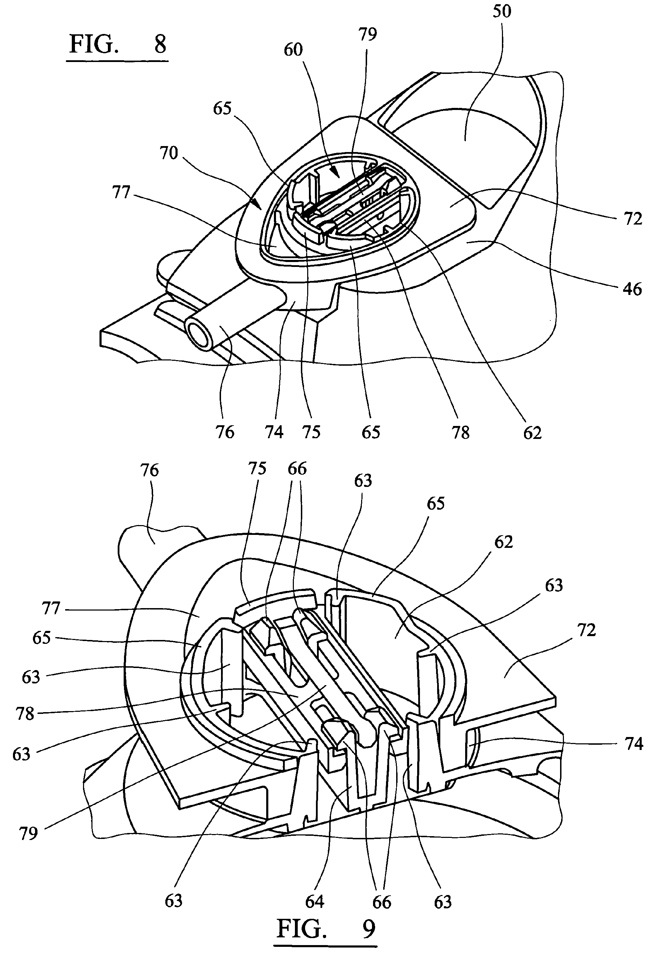

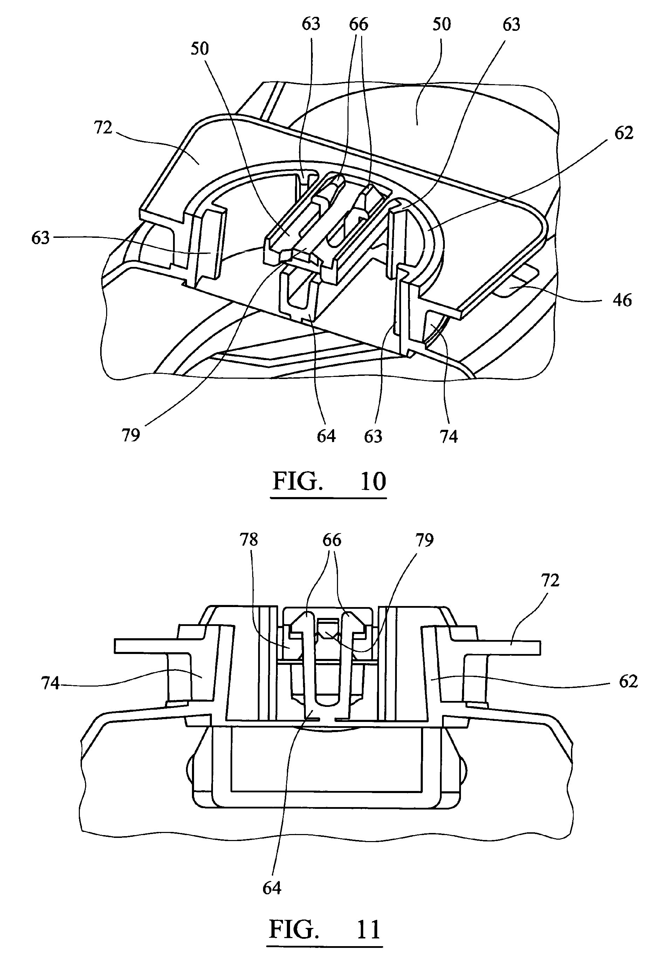

The reservoir bag assembly 20 comprises a flexible bag that is adapted to contain a reservoir of respiratory gas for inhalation by a patient. The flexible bag is therefore connected at one end to both the supply tube 10 and the respiratory mask 30. In particular, the reservoir bag assembly 20 includes a reservoir bag connector 70 for connecting the flexible bag to both the supply tube 10 and the respiratory mask 30. The reservoir bag connector 70 is described in more detail below in relation to Figures 8 to 11.

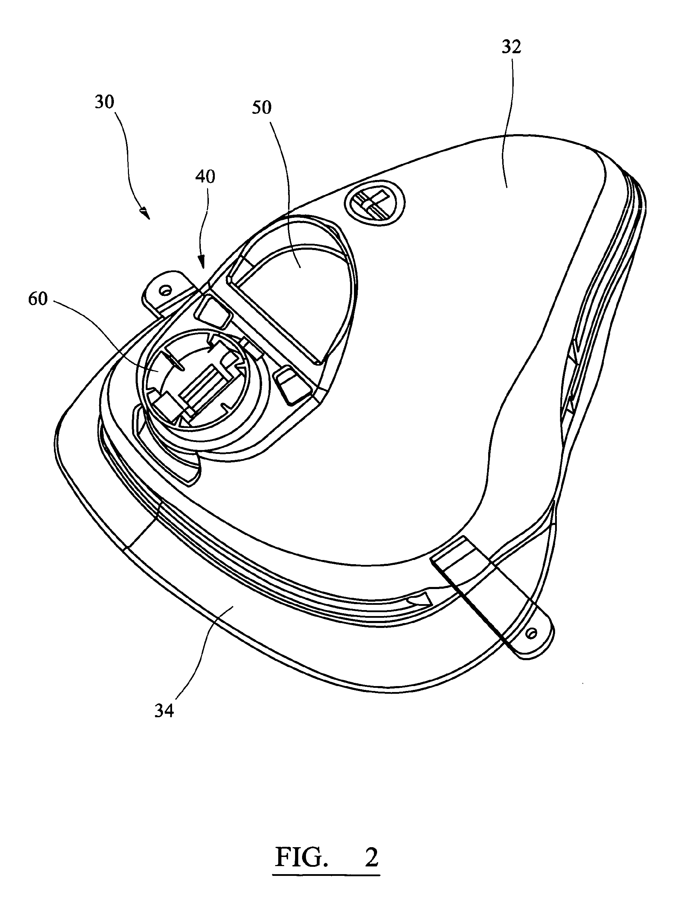

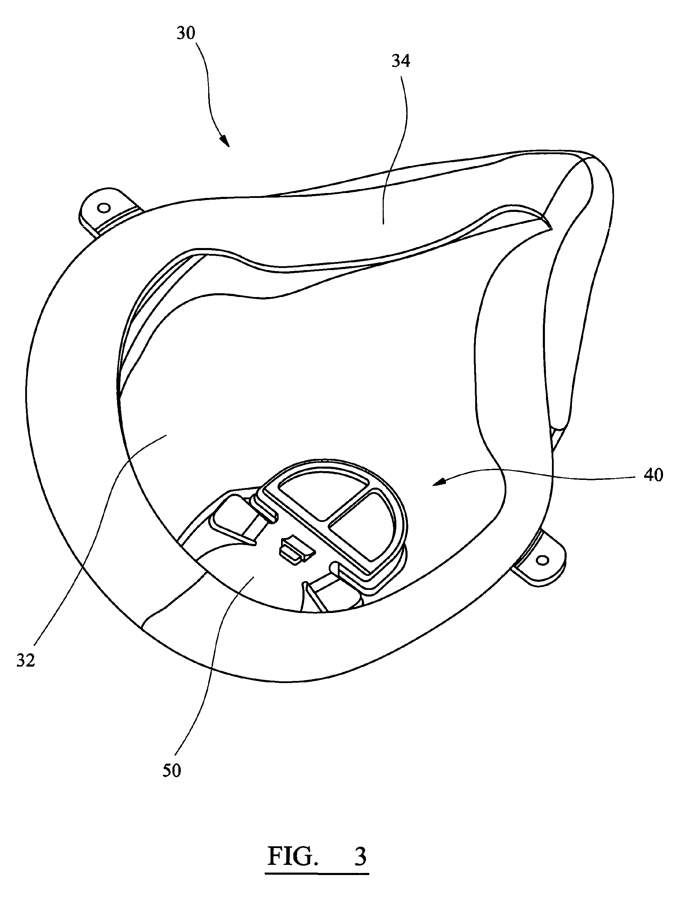

A first embodiment of the respiratory mask 30 is shown more clearly in Figures 2 and 3, and comprises a mask body 32, formed from a suitably strong and relatively rigid plastics material, such as polypropylene, and a sealing flange 34 formed from a suitable plastics material, such as a Styrene-Ethylene-Butylene-Styrene (SEBS)-based thermoplastic elastomer. The respiratory mask 30 is manufactured using a so-called two-shot injection moulding process. In particular, the mask body 32 is firstly injection moulded as a single component, and the sealing flange 34 is then injection moulded onto the surface of the mask body 32. Furthermore, in this embodiment of the invention, the sealing flange 34 has a configuration as described in published UK patent application GB 2412594.

The mask body 32 defines a cavity from which the respiratory gas is delivered to a patient, and comprises a mouth portion and a nose portion. At the mouth portion of the mask body 32, the respiratory mask 30 includes a valve assembly 40 that controls the passage of gases into and out of the cavity defined by the mask body 32.

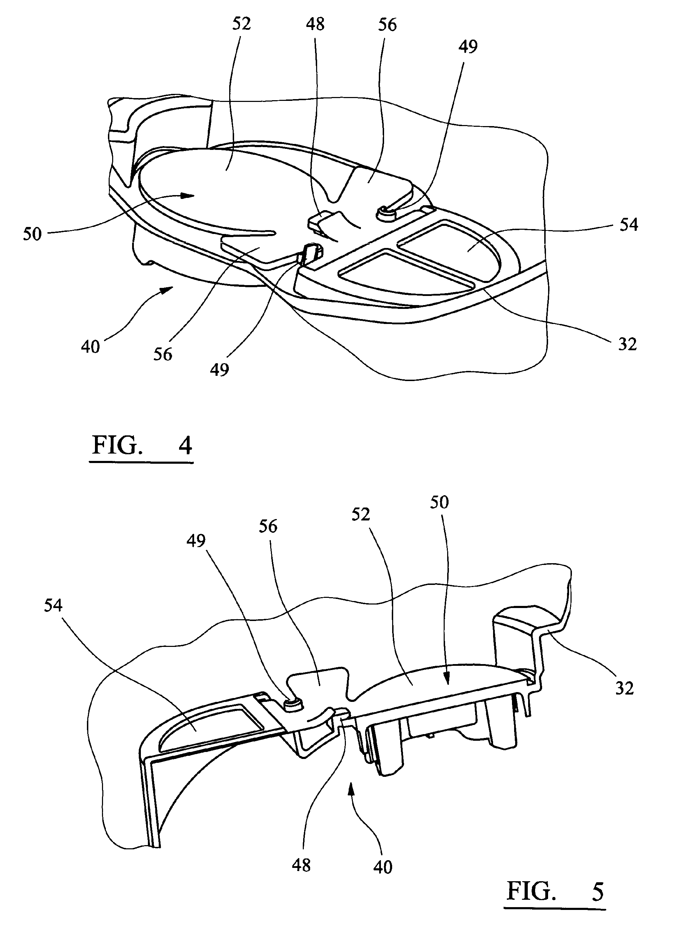

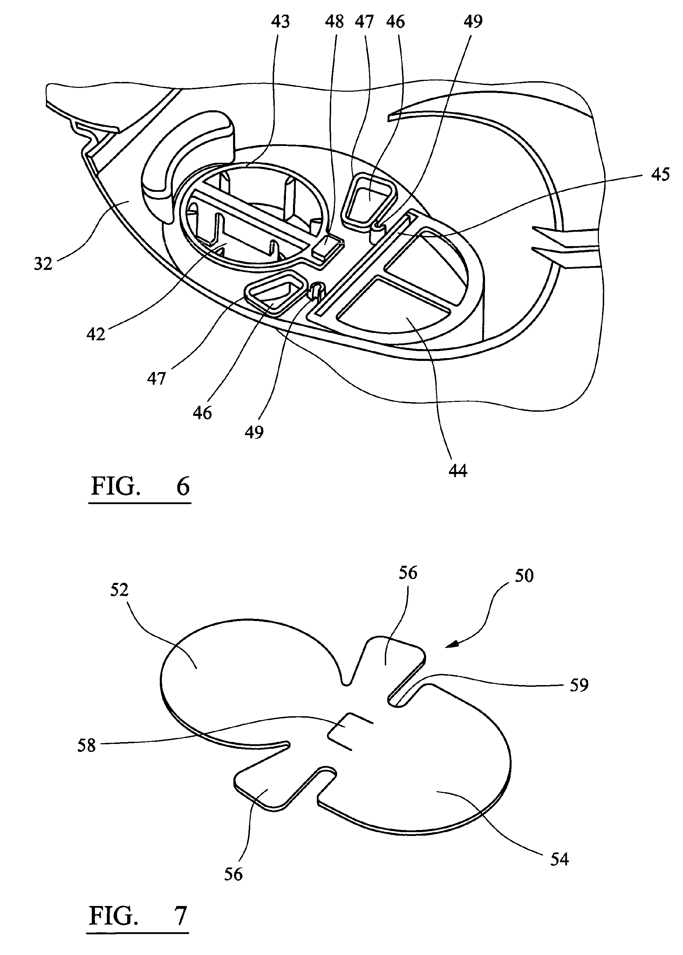

The valve assembly 40 is shown in more detail in Figures 4 and 5, and comprises a valve membrane 50 and valve formations formed integrally with the mask body 32. The valve formations and the valve membrane 50 are shown in isolation in Figures 6 and 7, respectively.

Referring now principally to Figure 6, the valve formations comprise an inhalation port 42, an exhalation port 44, a pair of safety ports 46, a retaining clip 48, and a pair of location posts 49.

The inhalation port 42 comprises a pair of semi-circular apertures in the wall of the mask body 32, and a raised valve seat 43 that surrounds the pair of apertures on the interior surface of the mask body 32. The raised valve seat 43 is circular in shape and has a flat operative surface. As shown more clearly in Figure 2, the inhalation port 42 includes a generally cylindrical connector 60 on the external surface of the mask body 32. The connector 60 is adapted to connect the inhalation port 42 to the reservoir bag assembly 20, as described in more detail below in relation to Figures 8 to 11.

The exhalation port 44 is situated within a generally semi-circular recess in the exterior surface of the mask body 32, and comprises a pair of apertures formed in the base of that recess. A mount 45 for the valve membrane 50 is also provided, which comprises a slot formed in the wall of the mask body 32 such that the membrane 50 extends through the wall of the mask body 32, and a shoulder upon which the membrane 50 rests so as to prevent the passage of gas through the slot during use. The mount 45 is arranged adjacent, and parallel, to the straight edge of the semi-circular recess. In addition, the pair of apertures of the exhalation port 44 are surrounded by a continuous valve seat defined by an external surface of the mask body 32 at the base of the semi-circular recess.

The valve seats of the inhalation and exhalation ports 42,44 are orientated in substantially the same plane, and the mount 45, retaining clip 48 and location posts 49 together act to retain the membrane 50 within the valve assembly during use.

The inhalation and exhalation ports 42,44 are arranged along a central axis of the mask body 32. On each side of this central axis, between the inhalation and exhalation ports 42,44, a safety port 46 is provided. Each safety port 46 comprises a generally trapezoidal aperture in the mask body 32 surrounded by a continuous valve seat 47 that is inclined slightly relative to the plane of the inhalation and exhalation ports 42,44. In particular, the valve seats 47 of the safety ports 46 gradually increase in height as the valve seats 47 extend away from the central axis of the mask body 32.

The retaining clip 48 is L-shaped, with an inner member that projects perpendicularly from the interior surface of the mask body 32 between the safety ports 46, and an outer member that is orientated parallel to the adjacent surface of the mask body 32 and extends towards the inhalation port 42. The location posts 49 project perpendicularly from the interior surface of the mask body 32, and are each positioned between a safety port 46 and the mount 45.

The valve membrane 50 is shown in isolation in Figure 7, and comprises an inhalation member 52, an exhalation member 54, a pair of safety members 56, a central flap 58, and a waist 59 of reduced width relative to the adjacent parts of the membrane 50. The valve membrane 50 is a flat sheet of plastics material, which is formed by press cutting a larger sheet of plastics material, the flap 58 being formed by cutting the membrane 50 along a generally C-shaped line. The flap 58 is therefore deformable independently of the remainder of the membrane 50.

As shown most clearly in Figures 4 and 5, the retaining clip 48 extends through the opening in the valve membrane 50 that is formed by the flap 58, and the outer member of the retaining clip 48 extends over a surface of the membrane 50 that faces away from the interior surface of the mask body 32. In this configuration, the flap 48 rests upon the upper surface of the retaining clip 48, and the location posts 49 are located immediately adjacent to, and on each side of, the waist 59 of the membrane 50. The location posts 49 act to prevent movement of the membrane 50 along axes parallel to the surface of the membrane 50, and hence maintain engagement between the retaining clip 48 and the membrane 50. The mount 45, retaining clip 48 and location posts 49 therefore cooperate to fasten a centre portion of the membrane 50 to the mask body 32.

The inhalation member 52 of the valve membrane 50 is generally circular in shape, and is adapted to occlude the apertures of the inhalation port 42, and engage the corresponding valve seat 43 on the interior surface of the mask body 32, in a rest configuration.

The safety members 56 of the valve membrane 50 extend from a central portion of the valve membrane 50 that is interposed between the inhalation and exhalation members 52,54. The safety members 56 are each generally trapezoidal in shape, and are adapted to occlude the apertures of the safety ports 46, and engage the corresponding valve seats 47 on the interior surface of the mask body 32, in a rest configuration.

The exhalation member 54 of the valve membrane 50 is generally semicircular in shape. In the valve assembly 40, the valve membrane 50 extends over the shoulder, and through the opening, of the mount 45, and projects across the exterior surface of the mask body 32 within the semi-circular recess. The exhalation member 54 is therefore adapted to occlude the apertures of the exhalation port 44, and engage the corresponding valve seat on the exterior of the mask body 32, in a rest configuration.

At rest, the inhalation and exhalation members 52,54 of the valve membrane 50 are arranged within the same plane and engage the parallel valve seats of the inhalation and exhalation ports 42,44, respectively. However, the safety members 56 rest upon the inclined valve seats 47 surrounding the apertures of the safety ports 46, and are therefore inclined relative to the remainder of the valve membrane 50.

The inhalation member 52 of the valve membrane 50 is arranged such that a reduction in the pressure within the cavity of the mask body 32 relative to the pressure within the connector 60, during inspiration by the patient, causes the inhalation member 52 of the valve membrane 50 to pivot about the retaining clip 48 away from the valve seat 43 of the inhalation port 42, so that respiratory gas is drawn into the cavity of the mask body 32 through the apertures of the inhalation port 42. The inhalation member 52 of the valve membrane 50 is also arranged such that an increase in the pressure within the cavity of the mask body 32 relative to the pressure within the connector 60, during expiration by a patient, causes the inhalation member 52 of the valve membrane 50 to pivot about the retaining clip 48 into engagement with the valve seat 43 of the inhalation port 42, so that exhaled gases are not able to escape from the cavity of the mask body 32 through the apertures of the inhalation port 42. In the event that the pressure within the cavity of the mask body 32 is less than the pressure within the connector 60, during expiration by the patient, respiratory gas will enter the cavity of the mask body 32, but exhaled gases will not flow through the inhalation port 42 due to the adverse pressure gradient.

The exhalation member 54 of the valve membrane 50 is arranged such that an increase in the pressure within the cavity of the mask body 32 relative to atmospheric pressure, during expiration by a patient, causes the exhalation member 54 of the valve membrane 50 to pivot about the shoulder of the mount 45 away from the valve seat of the exhalation port 44, so that exhaled gases are able to escape into the atmosphere from the cavity of the mask body 32, through the apertures of the exhalation port 44. The exhalation member 54 of the valve membrane 50 is also arranged such that a reduction in the pressure within the cavity of the mask body 32 relative to atmospheric pressure, during inspiration by a patient, causes the exhalation member 54 of the valve membrane 50 to pivot about the shoulder of the mount 45 into engagement with the valve seat of the exhalation port 44, so that respiratory gas is unable to escape into the atmosphere from the cavity of the mask body 32, through the apertures of the exhalation port 42.

Since the safety members 56 are inclined relative to the remainder of the valve membrane 50, the resilient nature of the valve membrane 50 will resist any deformation of the safety members 56 away from the inclined valve seats 47 with a greater force than that resisting deformation of the inhalation member 52 during inspiration by the patient. The safety members 56 are therefore arranged so that, during normal operation, they will remain in engagement with the valve seats 47 of the safety ports 46, such that atmospheric air is not drawn into the cavity of the mask body 32 during inspiration by the patient. However, in the event that the oxygen supply fails, the pressure within the cavity of the mask body 32 will reduce relative to atmospheric pressure until the reduction in pressure is sufficient to cause deformation of the safety members 56 of the valve membrane 50 away from the valve seats 47 of the safety ports 46, so that gas is drawn from the atmosphere into the cavity of the mask body 32 through the apertures of the safety ports 46.

Turning now to Figures 8 to 11, which each show the respiratory mask connector 60 and the reservoir bag connector 70 in a locked configuration, with the flexible bag omitted for clarity.

The respiratory mask connector 60 comprises a tubular member 62 that projects from the exterior surface of the mask body 32 about the periphery of the inhalation port 42, and a cross-member 64 that extends across a diameter of the tubular member 62, immediately adjacent to the apertures of the inhalation port 42, along a central axis of the mask body 32.

The tubular member 62 includes two recesses in its outer end that accommodate part of the reservoir bag connector 70 in the locked configuration, as described in more detail below, and also six longitudinal strengthening ribs 63 on its interior surface.

The cross-member 64 has a U-shaped cross-section with its arms extending away from the apertures of the inhalation port 42, and being accommodated within the tubular member 62. The arms of the cross-member 64 are generally about one half of the height of the tubular member 62, save for an extended portion at each end of each arm of the cross-member 64. These extended portions define a pair of adjacent locking arms 66 at each end of the cross-member 64.

Each locking arm 66 includes an enlarged head that projects generally perpendicularly away from the adjacent locking arm 66, such that each locking arm 66 includes a surface that faces an aperture of the inhalation port 42. The enlarged head of each locking arm 66 has a tapered and rounded end.

The reservoir bag connector 70 comprises a flange 72 that is heat-bonded or affixed, with glue for example, to an exterior surface of the flexible bag that surrounds an aperture at one end of the flexible bag. In the locked configuration, as shown in Figures 8-11, the flange 72 extends over, but is separated from, the safety ports 46. The flange 72 therefore protects the safety ports 46 from accidental damage and/or occlusion during use.

The reservoir bag connector 70 further comprises a tubular supply connector 76 that extends along a longitudinal axis of the connector 70 towards the chin of the patient when fitted. The supply connector 76 is adapted for connection with a tubular connector of the small-bore supply tube 10. The reservoir bag connector 70 also includes a fluid passageway that leads between the supply connector 76, and a supply aperture 77 that is within the confines of the flange 72 and hence in communication with the aperture of the flexible bag. In this way, respiratory gas is supplied through the supply tube 10, the supply connector 76 and the supply aperture 77 into the flexible bag of the reservoir bag assembly 20, during use.

The reservoir bag connector 70 also comprises a cylindrical sleeve member 74 that extends from the inner edge of the flange 72, adjacent to the supply aperture 77, and is adapted to receive the tubular member 62 of the respiratory mask connector 60 with a close fit.

When the respiratory mask and reservoir bag connectors 60,70 are connected together in the locked configuration, the majority of the outer end of the tubular member 62 is flush with the outer surface of the flange 72. However, the tubular member 62 includes a pair of projections 65 that are situated at each side of the recess in the tubular member 62 that is adjacent to the supply aperture 77. In addition, the cylindrical sleeve member 74 includes a similar projection 75 that is interposed between the projections 65 of the tubular member 62. These projections 65,75 prevent the flexible bag from collapsing, during use, in such a way that gas is unable to pass between the inhalation port 42 and the flexible bag and/or the supply connector 76.

The reservoir bag connector 70 also includes a cross-member 78 that extends along the longitudinal axis of the connector 70 between the supply aperture 77 and a facing part of the flange 72. The cross-member 78 of the reservoir bag connector 70 is adapted to be received within the recesses in the tubular member 62 of the respiratory mask connector 60 and engage the locking arms 66 of that connector 60.

The cross-member 78 of the reservoir bag connector 70 comprises a pair of parallel L-shaped (in cross-section) members that together define a central opening with a ledge on either side, and a pair of resilient arms 79 that are mounted on a central bridge between the L-shaped members and extend in opposite directions along the longitudinal axis of the central opening.

The cross-member 78 of the reservoir bag connector 70, and the locking arms 66 of the respiratory mask connector 60, are adapted to engage one another as the tubular member 62 of the respiratory mask connector 60 is received within the sleeve member 74 of the reservoir bag connector 70. In particular, the enlarged heads of the locking arms 66 are urged against the underside of the L-shaped members of the cross-member 78 of the reservoir bag connector 70.

The tapered and rounded ends of the locking arms 66 are arranged so that the enlarged heads of each adjacent pair of locking arms 66 are deformed towards each other by this action, such that the enlarged heads of the locking arms 66 are then urged against the underside of the resilient arms 79. This causes the outer ends of the resilient arms 79 to be deformed away from the L-shaped members. When the surfaces of the locking arms 66 that face the inhalation port 42 reach the surfaces of the ledges on each side of the opening in the cross-member 78, the locking arms 66 will be able to return to their rest orientation. This will cause the enlarged heads of the locking arms 66 to engage the ledges of the cross-member 78 so as to attach the respiratory mask and respiratory bag connectors 60,70 together. This will also permit the resilient arms 79 to return to their rest configuration, such that the resilient arms 79 are now interposed between the enlarged heads of each pair of adjacent locking arms 66. The presence of the resilient arms 79 between the enlarged heads of the locking arms 66 prevents inward deformation of the locking arms 66, and hence release of the reservoir bag connector 70. The respiratory mask and respiratory bag connectors 60,70 are therefore locked together in this configuration.

Before use, the respiratory mask and respiratory bag connectors 60,70 are locked together, as discussed above, such that the respiratory mask assembly 20 is in communication with the inhalation port 42 of the respiratory mask 30. The supply tube 10 is then connected at one end to a supply of respiratory gas, and at the other end to the supply connector 76 of the respiratory bag connector 70.

Once the respiratory gas delivery apparatus has been assembled, the mask body 32 is secured to a patient's head such that the patient's nose and mouth are accommodated within the cavity defined by the mask body 32.

Respiratory gas is then continuously supplied to the flexible bag of the respiratory bag assembly 20 through the supply connector 76 and supply aperture of the respiratory bag connector 70. In particular, respiratory gas is supplied to the respiratory bag assembly 20 at a rate appropriate for maintaining a reservoir of respiratory gas within the flexible bag that is sufficient for meeting the patient's peak inspiratory demands, without becoming completely depleted.

During use, inhalation by the patient will reduce the pressure within the cavity of the mask body 32 relative to the pressure within the connector 60. This reduction in pressure will act to deform the inhalation member 52 of the valve membrane 50 away from the valve seat 43 of the inhalation port 42, so that respiratory gas is drawn from the reservoir bag assembly 20 into the cavity of the mask body 32 through the apertures of the inhalation port 42.

Exhalation by the patient will increase the pressure within the cavity of the mask body 32 relative to the pressure within the reservoir bag 20 and also atmospheric pressure. This increase in pressure will act to return the inhalation member 52 of the valve membrane 50 into engagement with the valve seat 43 of the inhalation port 42, such that exhaled gases will not enter the reservoir bag. In addition, this increase in pressure will act to deform the exhalation member 54 of the valve membrane 50 away from the valve seat of the exhalation port 44, so that exhaled gases are able to escape into the atmosphere from the cavity of the mask body 32, through the apertures of the exhalation port 44.

In the event that the supply of respiratory gas fails, or the inhalation port 42 becomes blocked, the pressure within the cavity of the mask body 32 will reduce relative to atmospheric pressure until the reduction in pressure is sufficient to cause deformation of the safety members 56 of the valve membrane 50 away from the valve seats 47 of the safety ports 46, so that gas is drawn from the atmosphere into the cavity of the mask body 32 through the apertures of the safety ports 46.

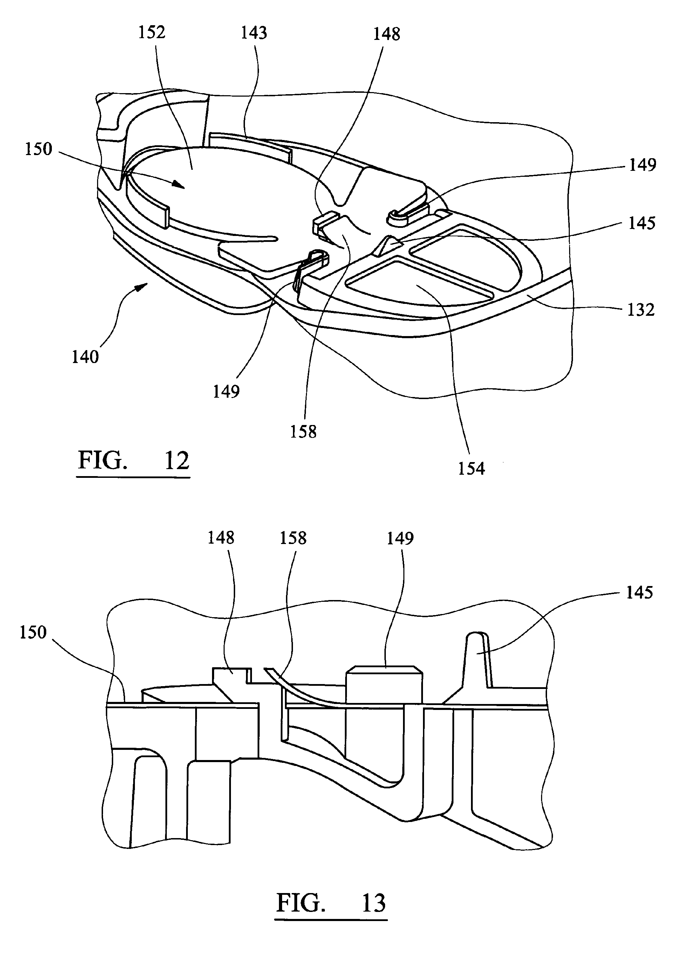

Figures 12 and 13 show a valve assembly 140 that forms part of a second embodiment of a respiratory mask according to the invention. The valve assembly 140 of the second embodiment is identical to the valve assembly 40 of the first embodiment, save for the inclusion of a modified retaining clip 148, modified location posts 149, a pair of retaining skirts 143 and an assembly tab 145.

In particular, the retaining clip 148 includes an additional upstanding projection at the distal end of its outer member, which acts to restrict, and preferably prevent, movement of the valve membrane 150 by providing a barrier for the flap 158 of the membrane. Furthermore, the retaining clip 148 includes a chamfered lower edge under which the membrane 150 is located during engagement with the valve formations, thereby facilitating that engagement.

The location posts 149 of this embodiment are of greater height than the location posts 49 of the first embodiment, and each of the location posts 149 includes an additional retaining wall. The additional retaining walls of the location posts 149 extend alongside the straight edges of the semi-circular exhalation member 154, on each side of the waist of the membrane 150. These features act to improve the engagement between the valve membrane 150 and the valve formations, and also restrict, and preferably prevent, rotation of the valve membrane 150 relative to the valve formations.

The retaining skirts 143 of the second embodiment are upstanding projections that extend about portions of the valve seat of the inhalation valve, such that the inhalation member 152 of the valve membrane 150 is closely received between the retaining skirts 143. The retaining skirts 143 therefore act to restrict, and preferably prevent, movement of the valve membrane 150 relative to the valve formations.

Finally, the assembly tab 145 projects upwardly from an interior surface of the mask body 132 between the apertures and the mount of the exhalation port. Since the valve seat of the exhalation port is defined by an external surface of the mask body 132 surrounding the apertures, the assembly tab 145 will not interfere with correct assembly of the valve membrane 150 within the valve assembly 140. However, if the valve membrane 150 is incorrectly engaged with the valve formations, such that the valve membrane 150 does not extend though the mount to the exterior of the mask body 132, then the assembly tab 145 will prevent engagement of the valve membrane 150 with the location posts 149. This feature therefore prevents an assembled valve configuration in which the exhalation member 154 of the valve membrane 150 is situated on the interior of the mask body 132, and hence in which the exhalation valve would not open during exhalation of the patient.

## Claims

1. Respiratory gas delivery apparatus comprising a respiratory mask (30) and a reservoir bag assembly (20), the respiratory mask (30) comprising a mask body (32,132) defining a cavity and being adapted to fit about the mouth and/or nose of the patient such that respiratory gas can be inhaled by the patient from the cavity, and an inhalation port (42) for enabling passage of gas into the cavity of the mask body (32,132), and the reservoir bag assembly (20) comprising a flexible bag for containing a reservoir of respiratory gas, **characterised in that** the respiratory mask (30) and the reservoir bag assembly (20) being provided with corresponding connectors (60,70) adapted for engagement so as to enable fluid communication between the flexible bag and the respiratory mask (30), wherein said connectors (60,70) are provided with formations (66,78) adapted to interlock upon engagement of the reservoir bag assembly (20) with the respiratory mask (30).

2. Respiratory gas delivery apparatus as claimed in Claim 1, wherein the interlocking formations (66,78) of the reservoir bag assembly (20) and the respiratory mask (30) are adapted to be brought into interlocking engagement by relative movement of the corresponding connectors (60,70) during engagement of those connectors (60,70).

3. Respiratory gas delivery apparatus as claimed in Claim 2, wherein the interlocking formations (66,78) are adapted to require the connectors (60,70) to have a particular orientation relative to each other, and the connectors (60,70) include guide formations that limit the relative orientation of the connectors (60,70) during engagement.

4. Respiratory gas delivery apparatus as claimed in any preceding claim, wherein the interlocking formations (66,78) of one component of the respiratory gas delivery apparatus, either the respiratory mask (30) or the reservoir bag assembly (20), comprises a retaining arm (66) adapted to engage a corresponding detent of the other component, such that the retaining arm (66) and the corresponding detent (78) act to prevent disengagement of the corresponding connectors (60,70).

5. Respiratory gas delivery apparatus as claimed in Claim 4, wherein each retaining arm (66) is adapted to be deformed from a rest configuration during engagement of the corresponding connectors (60,70) by means of a guide surface of the corresponding connector, and the guide surface is adapted so that the deformed retaining arm (66) then travels along the guide surface until the retaining arm (66) is able to resiliently engage the corresponding detent (78).

6. Respiratory gas delivery apparatus as claimed in Claim 5, wherein the deformed retaining arm (66) travels along the guide surface during engagement of the connectors (60,70) until corresponding operative surfaces of the retaining arm (66) and the detent (78) are aligned, and each deformed retaining arm (66) has sufficient resilience that when the operative surfaces are aligned, the deformed retaining arm (66) is able to move back towards its rest configuration, such that the operative surfaces are brought into engagement.

7. Respiratory gas delivery apparatus as claimed in any preceding claim, wherein the interlocking formations (66,78) of the respiratory mask (30) and the reservoir bag assembly (20) are adapted such that disengagement of these components is not possible without breaking those formations (66,78).

8. Respiratory gas delivery apparatus as claimed in Claim 7, wherein the respiratory mask (30) and/or the reservoir bag assembly (20) includes a locking member (79) that prevents disengagement of the interlocking formations (66,78), and the locking member (79) is adapted to prevent deformation of one or more retaining arms (66) out of engagement with one or more corresponding detents (78).

9. Respiratory gas delivery apparatus as claimed in Claim 8, wherein the locking member (79) includes one or more resilient arms that are adapted to be deformed away from a rest configuration by a retaining arm (66) that has itself been deformed during the engagement action, adapted to return to their rest configuration when the deformed retaining arm (66) moves into engagement with a corresponding detent (78), and hence no longer acts upon the one or more resilient arms, and adapted to then become situated adjacent to the previously deformed retaining arm (66) so as to prevent disengagement of the retaining arm (66) from the corresponding detent (78).

10. Respiratory gas delivery apparatus as claimed in any preceding claim, wherein the connectors (60,70) of the respiratory mask (30) and the reservoir bag assembly (20) each include a tubular connecting member (62,74) adapted to engage each other so as to define a fluid conduit between the inhalation port (42) of the respiratory mask (30) and the flexible bag of the reservoir bag assembly (20), and the interlocking formations (66,78) of the respiratory mask (30) and the reservoir bag assembly (20) are accommodated within their associated tubular connecting members (62,74), so that the interlocking formations (66,78) are brought into interlocking engagement as one tubular connecting member (62,74) is received within the other tubular connecting member (62,74).

11. Respiratory gas delivery apparatus as claimed in any preceding claim, wherein the connector (70) of the reservoir bag assembly (20) and/or the connector (60) of the respiratory mask (30) includes one or more safety projections (65,75) that project beyond an entrance port into the flexible bag and at least partially define a discontinuous surface upon which the flexible bag rests when collapsed, the safety formations (65,75) ensuring that gas is able to pass between the interior of the flexible bag and the entrance port in the event that the flexible bag has collapsed across the entrance port, during use.

12. A reservoir bag assembly (20) for use with a respiratory mask (30), the reservoir bag assembly (20) comprising a flexible bag for containing a reservoir of respiratory gas, and a connector (70) adapted for engagement with a corresponding connector (60) of the respiratory mask (30) so as to enable fluid communication between the flexible bag and the respiratory mask (30), wherein the connector (70) of the reservoir bag assembly (20) includes formations (66,78) adapted to interlock with corresponding formations (66,78) of the connector (60) of the respiratory mask (30) upon engagement of the reservoir bag assembly (20) with the respiratory mask (30).

13. A reservoir bag as claimed in Claim 12, wherein the flexible bag is formed by welding or otherwise joining two sheets of synthetic plastics material.

14. A reservoir bag as claimed in Claim 12 or Claim 13, wherein the connector (70) of the reservoir bag assembly (20) includes a mount (72) to which the flexible bag is bonded, the mount (72) defining an entrance port leading into the interior of the flexible bag, and the mount (72) being adapted to cover, but be separated from, one or more safety ports (46) of the respiratory mask (30).

15. A respiratory mask (30) for use with a reservoir bag assembly (20), the respiratory mask (30) comprising a mask body (32,132) defining a cavity and being adapted to fit about the mouth and/or nose of the patient such that respiratory gas can be inhaled by the patient from the cavity, an inhalation port (42) for enabling passage of gas into the cavity of the mask body (32,132), and a connector (60) adapted for engagement with a corresponding connector (70) of the reservoir bag assembly (20) so as to enable fluid communication between the inhalation port (42) and the reservoir bag assembly (20), wherein the connector (60) of the respiratory mask (30) includes formations (66,78) adapted to interlock with corresponding formations (66,78) of the connector (70) of the reservoir bag assembly (20) upon engagement of the respiratory mask (30) with the reservoir bag assembly (20).

16. A respiratory mask (30) as claimed in Claim 15, wherein the inhalation port (42) and the connector (60) of the respiratory mask (30) are formed integrally with the mask body (32,132), such that the mask body (32,132) including the inhalation port (42) and the connector (60) is defined by a single component.

17. A respiratory mask (30) as claimed in Claim 16, wherein the mask body (32,132) including the inhalation port (42) and the connector (60) is formed as a single component from plastics material in an injection moulding process.

18. Respiratory gas delivery apparatus as claimed in Claim 1, wherein fluid communication between the flexible bag and the respiratory mask (30) is through an entrance port of the flexible bag, and wherein the connector of the reservoir bag assembly (20) and/or the connector of the respiratory mask (30) includes one or more safety projections (65,75) that project beyond the entrance port into the flexible bag and at least partially define a discontinuous surface upon which the flexible bag rests when collapsed.

19. Respiratory gas delivery apparatus as claimed in Claim 1, wherein the reservoir bag assembly (20) comprises a connector (70) adapted for engagement with a corresponding connector (60) of the respiratory mask (30) so as to enable fluid communication between the flexible bag and the respiratory mask (30) through an entrance port of the flexible bag, wherein the connector (70) of the reservoir bag assembly (20) includes one or more safety projections (65,75) that project beyond the entrance port into the flexible bag and at least partially define a discontinuous surface upon which the flexible bag rests when collapsed.

20. Respiratory gas delivery apparatus as claimed in Claim 1, wherein fluid communication is enabled between the inhalation port (42) and a flexible bag of the reservoir bag assembly (20) through an entrance port of the flexible bag, wherein the connector (60) of the respiratory mask (30) includes one or more safety projections (65,75) that project beyond the entrance port into the flexible bag and at least partially define a discontinuous surface upon which the flexible bag rests when collapsed.

21. Respiratory gas delivery apparatus as claimed in any one of Claims 18 to 20, wherein the connectors (60,70) of the reservoir bag assembly (20) and the respiratory mask (30) each include a tubular connecting member (62,74), and the corresponding tubular connecting members (62,74) are adapted to engage each other so as to define a fluid conduit between the inhalation port (42) and the entrance port.

22. Respiratory gas delivery apparatus as claimed in Claim 21, wherein the safety projections (65,75) of the reservoir bag assembly (20) and/or the respiratory mask (30) extend from the end of the associated tubular connecting member (62,74) that defines, and/or is situated adjacent to, the entrance port.

23. Respiratory gas delivery apparatus as claimed in Claim 22, wherein the safety projections (65,75) have rounded end surfaces so as to reduce the risk of the flexible bag becoming damaged by the safety projections (65,75) during use.

## Patentansprüche

1. Atemgaszufuhrvorrichtung, umfassend eine Atemmaske (30) und eine Vorratsbeutelanordnung (20), wobei die Atemmaske (30) einen Maskenkörper (32, 132), der einen Hohlraum definiert und derart ausgelegt ist, dass er auf den Mund und/oder die Nase des Patienten passt, sodass Atemgas von dem Patienten aus dem Hohlraum eingeatmet werden kann, und eine Inhalationsöffnung (42), welche das Strömen von Gas in den Hohlraum des Maskenkörpers (32, 132) ermöglicht, umfasst und die Vorratsbeutelanordnung (20) einen flexiblen Beutel zur Aufnahme eines Vorrats an Atemgas umfasst, **dadurch gekennzeichnet, dass**
die Atemmaske (30) und die
Vorratsbeutelanordnung (20) mit entsprechenden für die Ineingriffnahme ausgelegten Verbindungselementen (60, 70) versehen sind, um die Fluidkommunikation zwischen dem flexiblen Beutel und der Atemmaske (30) zu ermöglichen, wobei die Verbindungselemente (60, 70) mit Ausbildungen (66, 78) versehen sind, die derart ausgelegt sind, dass sie sich bei Ineingriffnahme der Vorratsbeutelanordnung (20) mit der Atemmaske (30) ineinander verriegeln.

2. Atemgaszufuhrvorrichtung nach Anspruch 1, wobei die sich ineinander verriegelnden Ausbildungen (66, 78) der Vorratsbeutelanordnung (20) und der Atemmaske (30) derart ausgelegt sind, dass sie in eine verriegelnde Ineingriffnahme durch die relative Bewegung der entsprechenden Verbindungselemente (60, 70) während der Ineingriffnahme dieser Verbindungselemente (60, 70) gebracht werden.

3. Atemgaszufuhrvorrichtung nach Anspruch 2, wobei die sich ineinander verriegelnden Ausbildungen (66, 78) derart ausgelegt sind, dass die Verbindungselemente (60, 70) eine bestimmte Ausrichtung zueinander aufweisen müssen, und die Verbindungselemente (60, 70) Führungsausbildungen umfassen, welche die relative Ausrichtung der Verbindungselemente (60, 70) während der Ineingriffnahme begrenzen.

4. Atemgaszufuhrvorrichtung nach einem vorhergehenden Anspruch, wobei die sich ineinander verriegelnden Ausbildungen (66, 78) einer Komponente der Atemgaszufuhrvorrichtung, entweder der Atemmaske (30) oder der Vorratsbeutelanordnung (20), einen Haltearm (66) umfassen, der derart ausgelegt ist, dass er in eine entsprechende Arretierung der anderen Komponente eingreift, sodass der Haltearm (66) und die entsprechende Arretierung (78) bewirken, dass eine Trennung der entsprechenden Verbindungselemente (60, 70) verhindert wird.

5. Atemgaszufuhrvorrichtung nach Anspruch 4, wobei jeder Haltearm (66) derart ausgelegt ist, dass er aus einer Ruhekonfiguration während der Ineingriffnahme der entsprechenden Verbindungselemente (60, 70) durch eine Führungsfläche des entsprechenden Verbindungselements verformt wird, und die Führungsfläche derart ausgelegt ist, dass sich der verformte Haltearm (66) dann entlang der Führungsfläche verschiebt, bis der Haltearm (66) in der Lage ist, die entsprechende Arretierung (78) elastisch in Eingriff zu nehmen.

6. Atemgaszufuhrvorrichtung nach Anspruch 5, wobei sich der verformte Haltearm (66) entlang der Führungsfläche während der Ineingriffnahme der Verbindungselemente (60, 70) verschiebt, bis entsprechende Funktionsflächen des Haltearms (66) und der Arretierung (78) ausgerichtet sind und jeder verformte Haltearm (66) ausreichend Elastizität aufweist, sodass, wenn die Funktionsflächen ausgerichtet sind, der verformte Haltearm (66) in der Lage ist, sich derart zurück in seine Ruhekonfiguration zu bewegen, dass die Funktionsflächen in Eingriff gebracht werden.

7. Atemgaszufuhrvorrichtung nach einem vorhergehenden Anspruch, wobei die sich ineinander verriegelnden Ausbildungen (66, 78) der Atemmaske (30) und der Vorratsbeutelanordnung (20) derart ausgelegt sind, dass eine Trennung dieser Komponenten nicht möglich ist, ohne diese Ausbildungen (66, 78) zu zerstören.

8. Atemgaszufuhrvorrichtung nach Anspruch 7, wobei die Atemmaske (30) und/oder die Vorratsbeutelanordnung (20) ein Sperrelement (79) umfassen, welches die Trennung der sich ineinander verriegelnden Ausbildungen (66, 78) verhindert, und das Sperrelement (79) derart ausgelegt ist, dass es die Verformung eines oder mehrerer Haltearme (66) aus dem Eingriff mit einer oder mehreren entsprechenden Arretierungen (78) heraus verhindert.

9. Atemgaszufuhrvorrichtung nach Anspruch 8, wobei das Sperrelement (79) einen oder mehrere elastische Arme umfasst, die derart ausgelegt sind, dass sie aus einer Ruhekonfiguration heraus durch einen Haltearm (66), der selbst während des Ineingriffnahmevorgangs verformt wurde, verformt werden, derart ausgelegt sind, dass sie in ihre Ruhekonfiguration zurückkehren, wenn sich der verformte Haltearm (66) in den Eingriff mit einer entsprechenden Arretierung (78) bewegt und somit nicht länger auf den einen oder die mehreren elastischen Arme wirkt, und derart ausgelegt sind, dass sie sich dann angrenzend an den zuvor verformten Haltearm (66) befinden, um eine Trennung des Haltearms (66) von der entsprechenden Arretierung (78) zu verhindern.

10. Atemgaszufuhrvorrichtung nach einem vorhergehenden Anspruch, wobei die Verbindungselemente (60, 70) der Atemmaske (30) und der Vorratsbeutelanordnung (20) jeweils ein röhrenförmiges Verbindungsglied (62, 74) umfassen, die beide derart ausgelegt sind, dass sie einander in Eingriff nehmen, um einen Fluidkanal zwischen der Inhalationsöffnung (42) der Atemmaske (30) und dem flexiblen Beutel der Vorratsbeutelanordnung (20) zu definieren, und die sich ineinander verriegelnden Ausbildungen (66, 78) der Atemmaske (30) und der Vorratsbeutelanordnung (20) in ihren zugehörigen röhrenförmigen Verbindungsgliedern (62, 74) untergebracht sind, sodass die sich ineinander verriegelnden Ausbildungen (66, 78) in einen verriegelnden Eingriff gebracht werden, wenn ein röhrenförmiges Verbindungsglied (62, 74) in dem anderen röhrenförmigen Verbindungsglied (62, 74) aufgenommen wird.

11. Atemgaszufuhrvorrichtung nach einem vorhergehenden Anspruch, wobei das Verbindungelement (70) der Vorratsbeutelanordnung (20) und/oder das Verbindungelement (60) der Atemmaske (30) einen oder mehrere Sicherheitsvorsprünge (65, 75) umfasst, die über eine Eintrittsöffnung hinaus in den flexiblen Beutel hervorstehen und zumindest teilweise eine diskontinuierliche Fläche definieren, auf welcher der flexible Beutel aufliegt, wenn er zusammengefallen ist, wobei die Sicherheitsausbildungen (65, 75) sicherstellen, dass Gas zwischen dem Inneren des flexiblen Beutels und der Eintrittsöffnung in dem Fall, dass der flexible Beutel über der Eintrittsöffnung zusammengefallen ist, bei der Verwendung strömen kann.

12. Vorratsbeutelanordnung (20) zur Verwendung mit einer Atemmaske (30), wobei die Vorratsbeutelanordnung (20) einen flexiblen Beutel zur Aufnahme eines Vorrats von Atemgas und ein Verbindungselement (70) umfasst, das für die Ineingriffnahme mit einem entsprechenden Verbindungselement (60) der Atemmaske (30) ausgelegt ist, um die Fluidkommunikation zwischen dem flexiblen Beutel und der Atemmaske (30) zu ermöglichen, wobei das Verbindungselement (70) der Vorratsbeutelanordnung (20) Ausbildungen (66, 78) umfasst, die derart ausgelegt sind, dass sie sich mit entsprechenden Ausbildungen (66, 78) des Verbindungselements (60) der Atemmaske (30) bei Ineingriffnahme der Vorratsbeutelanordnung (20) mit der Atemmaske (30) ineinander verriegeln.

13. Vorratsbeutel nach Anspruch 12, wobei der flexible Beutel durch Schweißen oder anderweitiges Fügen zweier Folien aus synthetischem Kunststoffmaterial hergestellt wird.

14. Vorratsbeutel nach Anspruch 12 oder Anspruch 13, wobei das Verbindungselement (70) der Vorratsbeutelanordnung (20) eine Halterung (72) umfasst, mit welcher der flexible Beutel verbunden ist, wobei die Halterung (72) eine Eintrittsöffnung definiert, welche ins Innere des flexiblen Beutels führt, und die Halterung (72) derart ausgelegt ist, dass sie eine oder mehrere Sicherheitsöffnungen (46) der Atemmaske (30) abdeckt, jedoch davon getrennt ist.

15. Atemmaske (30) zur Verwendung mit einer Vorratsbeutelanordnung (20), wobei die Atemmaske (30) einen Maskenkörper (32, 132), der einen Hohlraum definiert und derart ausgelegt ist, dass er auf den Mund und/oder die Nase des Patienten passt, sodass Atemgas von dem Patienten aus dem Hohlraum eingeatmet werden kann, eine Inhalationsöffnung (42), welche das Strömen von Gas in den Hohlraum des Maskenkörpers (32, 132) ermöglicht, und ein Verbindungselement (60), das zur Ineingriffnahme mit einem entsprechenden Verbindungselement (70) der Vorratsbeutelanordnung (20) ausgelegt ist, umfasst, um die Fluidkommunikation zwischen der Inhalationsöffnung (42) und der Vorratsbeutelanordnung (20) zu ermöglichen, wobei das Verbindungselement (60) der Atemmaske (30) Ausbildungen (66, 78) umfasst, die derart ausgelegt sind, dass sie sich mit entsprechenden Ausbildungen (66, 78) des Verbindungselements (70) der Vorratsbeutelanordnung (20) bei Ineingriffnahme der Atemmaske (30) mit der Vorratsbeutelanordnung (20) ineinander versiegeln.

16. Atemmaske (30) nach Anspruch 15, wobei die Inhalationsöffnung (42) und das Verbindungselement (60) der Atemmaske (30) in einem Stück mit dem Maskenkörper (32, 132) ausgebildet sind, sodass der Maskenkörper (32, 132), umfassend die Inhalationsöffnung (42) und das Verbindungselement (60), durch eine Einzelkomponente definiert ist.

17. Atemmaske (30) nach Anspruch 16, wobei der Maskenkörper (32, 132), umfassend die Inhalationsöffnung (42) und das Verbindungselement (60), als Einzelkomponente aus Kunststoff in einem Spritzgussverfahren hergestellt wird.

18. Atemgaszufuhrvorrichtung nach Anspruch 1, wobei die Fluidkommunikation zwischen dem flexiblen Beutel und der Atemmaske (30) durch eine Eintrittsöffnung des flexiblen Beutels erfolgt und wobei das Verbindungselement der Vorratsbeutelanordnung (20) und/oder das Verbindungselement der Atemmaske (30) einen oder mehrere Sicherheitsvorsprünge (65, 75) umfasst, die über die Eintrittsöffnung hinaus in den flexiblen Beutel hervorstehen und zumindest teilweise eine diskontinuierliche Fläche definieren, auf welcher der flexible Beutel aufliegt, wenn er zusammengefallen ist.

19. Atemgaszufuhrvorrichtung nach Anspruch 1, wobei die Vorratsbeutelanordnung (20) ein Verbindungselement (70) umfasst, das zur Ineingriffnahme mit einem entsprechenden Verbindungselement (60) der Atemmaske (30) ausgelegt ist, um die Fluidkommunikation zwischen dem flexiblen Beutel und der Atemmaske (30) durch eine Eintrittsöffnung des flexiblen Beutels zu ermöglichen, wobei das Verbindungselement (70) der Vorratsbeutelanordnung (20) einen oder mehrere Sicherheitsvorsprünge (65, 75) umfasst, die über die Eintrittsöffnung hinaus in den flexiblen Beutel hervorstehen und zumindest teilweise eine diskontinuierliche Fläche definieren, auf welcher der flexible Beutel aufliegt, wenn er zusammengefallen ist.

20. Atemgaszufuhrvorrichtung nach Anspruch 1, wobei Fluidkommunikation zwischen der Inhalationsöffnung (42) und einem flexiblen Beutel der Vorratsbeutelanordnung (20) durch eine Eintrittsöffnung des flexiblen Beutels ermöglicht wird, wobei das Verbindungselement (60) der Atemmaske (30) einen oder mehrere Sicherheitsvorsprünge (65, 75) umfasst, die über die Eintrittsöffnung hinaus in den flexiblen Beutel hervorstehen und zumindest teilweise eine diskontinuierliche Fläche definieren, auf welcher der flexible Beutel aufliegt, wenn er zusammengefallen ist.

21. Atemgaszufuhrvorrichtung nach einem der Ansprüche 18 bis 20, wobei die Verbindungselemente (60, 70) der Vorratsbeutelanordnung (20) und der Atemmaske (30) jeweils ein röhrenförmiges Verbindungsglied (62, 74) umfassen und die entsprechenden röhrenförmigen Verbindungsglieder (62, 74) derart ausgelegt sind, dass sie einander in Eingriff nehmen, um einen Fluidkanal zwischen der Inhalationsöffnung (42) und der Eintrittsöffnung zu definieren.

22. Atemgaszufuhrvorrichtung nach Anspruch 21, wobei sich die Sicherheitsvorsprünge (65, 75) der Vorratsbeutelanordnung (20) und/oder der Atemmaske (30) vom Ende des zugehörigen röhrenförmigen Verbindungsglieds (62, 74) aus erstrecken, welches die Eintrittsöffnung definiert und/oder sich daran angrenzend befindet.

23. Atemgaszufuhrvorrichtung nach Anspruch 22, wobei die Sicherheitsvorsprünge (65, 75) abgerundete Endflächen aufweisen, um die Gefahr zu verringern, dass der flexible Beutel durch die Sicherheitsvorsprünge (65, 75) bei der Verwendung beschädigt wird.

## Revendications

1. Appareil de fourniture de gaz respirable comprenant un masque respiratoire (30) et un ensemble poche à réservoir (20), le masque respiratoire (30) comportant un corps de masque (32, 132) définissant une cavité et étant adapté pour s'ajuster autour de la bouche et/ou du nez du patient de sorte que le gaz respirable puisse être inhalé par le patient à partir de la cavité, et un orifice d'inhalation (42) pour permettre le passage du gaz dans la cavité du corps (32, 132) du masque, et l'ensemble poche à réservoir (20) comportant une poche souple destinée à contenir un réservoir de gaz respirable , **caractérisé en ce que** le masque respiratoire (30) et l'ensemble poche à réservoir (20) sont munis de connecteurs correspondants (60, 70) adaptés pour s'accoupler de façon à permettre la communication fluide entre la poche souple et le masque respiratoire (30), lesdits connecteurs (60, 70) étant pourvus de parties conformées (66, 78) adaptées pour se verrouiller lors de l'accouplement de l'ensemble poche à réservoir (20) au masque respiratoire (30).

2. Appareil de fourniture de gaz respirable selon la revendication 1, lesdites parties conformées à verrouillage (66, 78) de l'ensemble poche à réservoir (20) et du masque respiratoire (30) étant adaptées pour être amenées en prise de verrouillage par le mouvement relatif des connecteurs correspondants (60, 70) pendant l'accouplement de ces connecteurs (60, 70).

3. Appareil de fourniture de gaz respirable selon la revendication 2, lesdites parties conformées à verrouillage (66, 78) étant adaptées pour imposer que les connecteurs (60, 70) aient une orientation particulière l'un par rapport à l'autre, et les connecteurs (60, 70) incluant des parties conformées guides qui limitent l'orientation relative des connecteurs (60, 70) pendant l'accouplement.

4. Appareil de fourniture de gaz respirable selon l'une quelconque des revendications précédentes, lesdites parties conformées à verrouillage (66, 78) d'un composant de l'appareil de fourniture de gaz respirable, soit le masque respiratoire (30) soit l'ensemble poche à réservoir (20), comprenant un bras de rétention (66) adapté pour enclencher un cran correspondant de l'autre composant, de sorte que le bras de rétention (66) et le cran correspondant (78) agissent pour empêcher le désaccouplement des connecteurs correspondants (60, 70).

5. Appareil de fourniture de gaz respirable selon la revendication 4, chaque bras de rétention (66) étant adapté pour se déformer à partir d'une configuration au repos pendant l'accouplement des connecteurs correspondants (60, 70) au moyen d'une surface guide du connecteur correspondant, et la surface guide étant adaptée de sorte que le bras de rétention (66) déformé se déplace ensuite le long de la surface guide jusqu'à ce que le bras de rétention (66) soit capable d'enclencher de manière élastique le cran correspondant (78).

6. Appareil de fourniture de gaz respirable selon la revendication 5, ledit bras de rétention (66) déformé se déplaçant le long de la surface guide pendant l'accouplement des connecteurs (60, 70) jusqu'à ce que les surfaces actives correspondantes du bras de rétention (66) et du cran (78) soient alignées, et chaque bras de rétention (66) déformé ayant une résilience suffisante afin que lorsque les surfaces actives sont alignées, le bras de rétention (66) déformé soit capable de revenir dans sa configuration au repos, de sorte que les surfaces actives soient mises en prise.

7. Appareil de fourniture de gaz respirable selon l'une quelconque des revendications précédentes, lesdites parties conformées à verrouillage (66, 78) du masque respiratoire (30) et de l'ensemble poche à réservoir (20) étant adaptées de sorte que le désaccouplement de ces composants ne soit pas possible sans la rupture de ces parties conformées (66, 78).

8. Appareil de fourniture de gaz respirable selon la revendication 7, ledit masque respiratoire (30) et/ou ledit ensemble poche à réservoir (20) incluant un élément de verrouillage (79) qui empêche le désaccouplement des parties conformées à remouillage (66, 78), et ledit élément de remouillage (79) étant adapté pour empêcher la déformation d'un ou plusieurs bras de rétention (66) hors de prise avec un ou plusieurs crans correspondants (78).

9. Appareil de fourniture de gaz respirable selon la revendication 8, ledit élément de verrouillage (79) incluant un ou plusieurs bras résilients qui sont adaptés pour être déformés à l'écart d'une configuration au repos par un bras de rétention (66) qui a lui-même était déformé pendant l'opération d'accouplement, adaptés pour revenir dans leur configuration au repos lorsque le bras de rétention (66) déformé se met en prise avec un cran correspondant (78), et n'agissant donc plus sur lesdits un ou plusieurs bras résilients, et adaptés pour ensuite venir se placer adjacents au bras de rétention (66) précédemment déformé de façon à empêcher le désaccouplement du bras de rétention (66) du cran correspondant (78).

10. Appareil de fourniture de gaz respirable selon l'une quelconque des revendications précédentes, lesdits connecteurs (60, 70) du masque respiratoire (30) et de l'ensemble poche à réservoir (20) incluant chacun un élément de connexion tubulaire (62, 74) adapté pour s'accoupler l'un à l'autre de façon à définir un conduit de fluide entre l'office d'inhalation (42) du masque respiratoire (30) et la poche souple de l'ensemble poche à réservoir (20), et lesdites parties conformées à verrouillage (66, 78) du masque respiratoire (30) et de l'ensemble poche à réservoir (20) étant reçues à l'intérieur de leurs éléments de connexion tubulaires associés (62, 74), de sorte que les parties conformées à verrouillage (66, 78) soient amenées en prise de verrouillage étant donné qu'un élément de connexion tubulaire (62, 74) est reçu dans l'autre élément de connexion tubulaire (62, 74).

11. Appareil de fourniture de gaz respirable selon l'une quelconque des revendications précédentes, ledit connecteur (70) de l'ensemble poche à réservoir (20) et/ou le connecteur (60) du masque respiratoire (30) incluant une ou plusieurs parties saillantes de sécurité (65, 75) qui font saille au-delà d'un orifice d'entrée dans la poche souple et définissent au moins en partie une surface discontinue sur laquelle la poche souple repose lorsqu'elle est dégonflée, les parties conformées de sécurité (65, 75) garantissant que le gaz est capable de passer entre l'intérieur de la poche souple et l'orifice d'entrée dans le cas où la poche souple s'est dégonflée en travers de l'orifice d'entrée, en cours d'utilisation.

12. Ensemble poche à réservoir (20) destiné à être utilisé avec un masque respiratoire (30), l'ensemble poche à réservoir (20) comprenant une poche souple destinée à contenir un réservoir de gaz respirable, et un connecteur (70) adapté pour s'accoupler à un connecteur correspondant (60) du masque respiratoire (30) de façon à permettre la communication fluide entre la poche souple et le masque respiratoire (30), ledit connecteur (70) de l'ensemble poche à réservoir (20) incluant des parties conformées (66, 78) adaptées pour se verrouiller avec les parties conformées correspondantes (66, 78) du connecteur (60) du masque respiratoire (30) lors de l'accouplement de l'ensemble poche à réservoir (20) au masque respiratoire (30).

13. Poche à réservoir selon la revendication 12, ladite poche souple étant formée par soudage ou par ailleurs en joignant deux feuilles de matière plastique synthétique.

14. Poche à réservoir selon la revendication 12 ou 13, ledit connecteur (70) de l'ensemble poche à réservoir (20) incluant un support (72) auquel la poche souple est fixée, le support (72) définissant un orifice d'entrée conduisant à l'intérieur de la poche souple, et le support (72) étant adapté pour recouvrir un ou plusieurs orifices de sécurité (46) du masque respiratoire (30), mais en être séparé.

15. Masque respiratoire (30) destiné à être utilisé avec un ensemble poche à réservoir (20), le masque respiratoire (30) comprenant un corps de masque (32, 132) définissant une cavité et étant adapté pour s'ajuster autour de la bouche et/ou du nez du patient de sorte que le gaz respirable puisse être inhalé par le patient à partir de la cavité, un orifice d'inhalation (42) pour permettre le passage du gaz dans la cavité du corps (32, 132) du masque, et un connecteur (60) adapté pour s'accoupler à un connecteur correspondant (70) de l'ensemble poche à réservoir (20) de façon à permettre la communication fluide entre l'orifice d'inhalation (42) et l'ensemble poche à réservoir (20), ledit connecteur (60) du masque respiratoire (30) incluant des parties conformées (66, 78) adaptées pour se verrouiller avec les parties conformées correspondantes (66, 78) du connecteur (70) de l'ensemble poche à réservoir (20) lors de l'accouplement du masque respiratoire (30) à l'ensemble poche à réservoir (20).

16. Masque respiratoire (30) selon la revendication 15, ledit orifice d'inhalation (42) et le connecteur (60) du masque respiratoire (30) étant intégralement formés avec le corps (32, 132) du masque, de sorte que le corps (32, 132) du masque comportant l'orifice d'inhalation (42) et le connecteur (60) soit défini par un unique composant.

17. Masque respiratoire (30) selon la revendication 16, ledit corps (32, 132) du masque incluant l'orifice d'inhalation (42) et le connecteur (60) étant formé sous la forme d'un unique composant à partir de matière plastique dans un processus de moulage par injection.

18. Appareil de fourniture de gaz respirable selon la revendication 1, ladite communication fluide entre la poche souple et le masque respiratoire (30) se faisant au travers d'un orifice d'entrée de la poche souple, et ledit connecteur de l'ensemble poche à réservoir (20) et/ou le connecteur du masque respiratoire (30) incluant une ou plusieurs parties saillantes de sécurité (65, 75) qui font saillie au-delà de l'orifice d'entrée dans la poche souple et définissent au moins en partie une surface discontinue sur laquelle la poche souple repose lorsqu'elle est dégonflée.

19. Appareil de fourniture de gaz respirable selon la revendication 1, ledit ensemble poche à réservoir (20) comprenant un connecteur (70) adapté pour s'accoupler à un connecteur correspondant (60) du masque respiratoire (30) de façon à permettre la communication fluide entre la poche souple et le masque respiratoire (30) au travers d'un orifice d'entrée de la poche souple, ledit connecteur (70) de l'ensemble poche à réservoir (20) incluant une ou plusieurs parties saillantes de sécurité (65, 75) qui font saillie au-delà de l'orifice d'entrée dans la poche flexible et définissent au moins en partie un surface discontinue sur laquelle la poche souple repose lorsqu'elle est dégonflée.

20. Appareil de fourniture de gaz respirable selon la revendication 1, ladite communication fluide étant activée entre l'orifice d'inhalation (42) et une poche souple de l'ensemble poche à réservoir (20) au travers d'un orifice d'entrée de la poche souple, ledit connecteur (60) du masque respiratoire (30) incluant une ou plusieurs parties saillantes de sécurité (65, 75) qui font saillie au-delà de l'orifice d'entrée dans la poche flexible et définissent au moins en partie une surface discontinue sur laquelle la poche souple repose lorsqu'elle est dégonflée.

21. Appareil de fourniture de gaz respirable selon l'une quelconque des revendications 18 à 20, lesdits connecteurs (60, 70) de l'ensemble poche à réservoir (20) et du masque respiratoire (30) incluant chacun un élément de connexion tubulaire (62, 74), et les éléments de connexion tubulaires correspondants (62, 74) étant adaptés pour s'accoupler l'un à l'autre entre l'orifice d'inhalation (42) et l'orifice d'entrée.

22. Appareil de fourniture de gaz respirable selon la revendication 21, lesdites parties saillantes de sécurité (65, 75) de l'ensemble poche à réservoir (20) et/ou du masque respiratoire (30) s'étendant de l'extrémité de l'élément de connexion tubulaire associé (62, 74) qui définit l'orifice d'entrée et/ou est placé adjacent à celui-ci.

23. Appareil de fourniture de gaz respirable selon la revendication 22, lesdites parties saillantes de sécurité (65, 75) possédant des surfaces d'extrémité arrondies pour réduire le risque d'endommagement de la poche souple par les parties saillantes de sécurité (65, 75) en cours d'utilisation.
